Europäisches Patentamt

European Patent Office

Office européen des brevets

(19)

(11) Veröffentlichungsnummer: **0 340 663**
**A2**

(12) **EUROPÄISCHE PATENTANMELDUNG**

(21) Anmeldenummer: 89107758.8

(22) Anmeldetag: 28.04.89

(51) Int. Cl.4: **C07C 51/487 , C07C 57/30 , C07C 59/64 , C07C 57/58 , C07D 209/82**

(30) Priorität: 02.05.88 DE 3814887

(43) Veröffentlichungstag der Anmeldung:
08.11.89 Patentblatt 89/45

(84) Benannte Vertragsstaaten:
AT BE CH DE ES FR GB GR IT LI LU NL SE

(71) Anmelder: MEDICE Chem.-Pharm. Fabrik Pütter GmbH & Co. KG
Kuhloweg 37-39
D-5860 Iserlohn/Westfalen(DE)

(72) Erfinder: Blaschke, Gottfried, Prof. Dr.
Vredenweg 18
D-4400 Münster(DE)
Erfinder: Schulte, Karl-Ernst, Prof. Dr. Dr.
Fliednerstrasse 11
D-4400 Münster(DE)

(74) Vertreter: Hansen, Bernd, Dr.rer.nat. et al
Hoffmann, Eitle & Partner Patentanwälte
Arabellastrasse 4 Postfach 81 04 20
D-8000 München 81(DE)

(54) Verfahren zur Gewinnung von Enantiomeren von 2-Aryl-propionsäuren.

(57) Ein Verfahren zur Gewinnung von Enantiomeren von 2-Aryl-propionsäuren durch Umsetzung eines racemischen Gemisches derselben mit einem Aminenantiomer, wobei man das Racemat der 2-Aryl-propionsäure mit einer optisch aktiven Form von threo-1-p-Nitrophenyl-2-aminopropan-1,3-diol in die diastereomeren Salze überführt, diese auftrennt und die so erhaltenen reinen Diastereomeren in die freien Säuren der enantiomeren Formen der 2-Aryl-propionsäure oder deren Salze überführt.

EP 0 340 663 A2

## Verfahren zur Gewinnung von Enantiomeren von 2-Aryl-propionsäuren

Die Erfindung betrifft ein Verfahren zur Gewinnung von Enantiomeren von 2-Aryl-propionsäuren durch Umsetzung eines racemischen Gemisches derselben mit einem Aminenantiomer.

Verschiedene 2-Aryl-propionsäuren finden in Form ihres racemischen Gemisches aufgrund ihrer analgetischen und antiphlogistischen Eigenschaften in der Rheumatherapie Verwendung. Der Wirkungsmechanismus dürfte über eine Hemmung der "Arachidonsäure-Kaskade" (Bildung von Prostaglandinen, Thromboxanen, Leukotrienen) ablaufen.

Die eingesetzten Gemische enthalten im allgemeinen beide Antipoden der 2-Aryl-propionsäuren, die aber nicht pharmakologisch gleichwertig sind. Trotzdem werden in der Therapie üblicherweise - Naproxen stellt hier eine Ausnahme dar, da es in der S-Form eingesetzt ist - noch immer die unaufgetrennten Gemische verwendet. Es besteht somit ein Bedürfnis nach einfachen Methodiken der Gewinnung der reinen Antipoden.

So fanden Adams und Mitarbeiter (J. Pharm. Pharmakol. 28, 256-257), daß z.B. das S(+)-Ibuprofen in vitro die Prostaglandin-Synthetase 160 mal stärker hemmt als das R(-)-Isomere. Inzwischen wurde tierexperimentell und am Menschen gezeigt, daß das S(+)-Ibuprofen der potentere Arzneistoff ist. Mit S(+)-Ibuprofen kann der gleiche therapeutische Effekt mit einer wesentlich geringeren Dosis als mit dem Racemat erzielt werden, gleichzeitig treten die bei der Applikation des Racemats in therapeutischen Dosen beobachteten Nebenwirkungen nicht oder wesentlich geringer auf.

Letztere werden offenbar durch das nicht oder nur geringfügig analgetisch bzw. antiphlogistisch wirksame R(-)-Ibuprofen verursacht. Die unterschiedlichen pharmakologischen Eigenschaften der beiden Antipoden des Ibuprofens finden u.a. auch in der unterschiedlichen Pharmakokinetik und Biotransformation ihren Ausdruck (F. Jamali u. Mitarbeiter, Pharmac. Res. 5, 44 (1988).

Die Gewinnung der optisch reinen 2-Aryl-propionsäuren in technischem Maßstab kann über eine stereospezifische Synthese des Arzneistoffes oder durch Auftrennung von Racemat erfolgen. Für eine stereospezifische Synthese käme u.a. eine Friedel-Crafts-Synthese für Ibuprofen, z.B. von Isobutylbenzol mit optisch aktiven Milchsäure-Derivaten (z.B. S(+)-Mesyl-Milchsäuremethylester) oder R(-)-Chlorpropionsäureisobutylester in Frage. Die Synthesen verlaufen infolge von Isomerisierungen im allgemeinen aber mit schlechten Ausbeuten.

Die Auftrennung des racemischen Gemisches von 2-Aryl-propionsäuren ist in der US-A-4209638 (Boots Company Ltd., Nottingham) beschrieben und erfolgt über eine fraktionierte Kristallisation der diastereomeren Salze, die die beiden Säuren mit einer optisch aktiven Base bilden. Als Base findet die L-Form von α-methyl-bzw. -aryl-substituierten Benzylaminen Verwendung, mit denen die S(+)- und R(-)-Formen der 2-Aryl-propionsäuren diastereomere Salze bilden, die in aliphatischen Kohlenwasserstoffen bzw. Gemischen aus diesen Kohlenwasserstoffen und Aromaten unterschiedliche Löslichkeit besitzen und dadurch voneinander getrennt werden können. Aus den reinen diastereomeren Salzen lassen sich die Säuren durch übliche Arbeitsweisen mit verdünnten Mineralsäuren gewinnen.

Dieses Verfahren findet aus verschiedenen Gründen keine praktische Verwendung. Das Verfahren ist durch notwendiges mehrmaligs Umkristallisieren nicht nur zeitaufwendig, sondern auch unwirtschaftlich: Es führt z.B. nur zu geringen Ausbeuten. Zudem sind die verwendeten Basen teuer und chemisch nicht stabil; sie lassen sich nur unter Verlusten rein zurückgewinnen. Andere optisch aktive Basen, wie Ephedrin, Pseudoephedrin, Norephedrin oder Norpseudoephedrin, geben mit typischen 2-Aryl-propionsäuren, z.B. mit S(+)- bzw. R(-)-Ibuprofen, keine kristallinen diastereomeren Salze.

Es wurde nun überraschenderweise gefunden, daß sich zur Trennung des Racemats der 2-Aryl-propionsäuren besonders gut der Aminoalkohol threo-1- p-Nitrophenyl-2-aminopropan-1,3-diol eignet. Dieser Aminoalkohol, der gut kristallisiert, gegen Sauerstoff und Säuren stabil und unter Normalbedingungen nicht racemisierbar ist, bildet mit S(+)- und R(-)-Antipoden diastereomere Salze, die sich aus einfachen organischen Lösungsmitteln bzw. Lösungsmittelgemischen leicht trennen lassen.

Die Aufgabe der vorliegenden Erfindung wird daher durch die Schaffung eines Verfahrens der eingangs genannten Art gelöst, das dadurch gekennzeichnet ist, daß man das Racemat der 2-Aryl-propionsäure mit einer optisch aktiven Form von threo-1-p-Nitrophenyl-2-aminopropan-1,3-diol, vorzugsweise der D(-)-Form, in die diastereomeren Salze überführt, diese auftrennt und die so erhaltenen reinen Diastereomeren in die freien Säuren der enantiomeren Formen der 2-Aryl-propionsäure oder deren Salze überführt.

Als 2-Aryl-propionsäuren, die im erfindungsgemäßen Verfahren angewandt werden können, sollen besonders Flurbiprofen, Fenoprofen, Ketoprofen, Naproxen, Benoxaprofen und Ibuprofen genannt werden.

. Der Einsatz des vorstehend genannten Aminoalkohols führt zur Bildung diastereoisomerer Salze, die sich in organischen Lösungsmitteln stark unterschiedlich lösen. Dadurch ist es möglich, die optischen Antipoden z.B. durch Kristallisation zu trennen. Geeignete Lösungsmittel können durch den Fachmann durch einfache Versuche ermittelt werden. Üblicherweise handelt es sich um unpolare Lösungsmittel, denen ein Anteil an polarem Lösungsmittel zugefügt sein kann. Als solche unpolaren Lösungsmittel können aliphatische, alicyclische und aromatische Kohlenwasserstoffe, z.B. Petroletherfraktionen (Siedepunkt: 60-90 °C), Cyclohexan etc. genannt werden, während die polare Komponente mit Vorzug z.B. einen niedrigen, vorzugsweise aliphatischen Alkohol darstellen kann. Als solche könnte Methanol, Ethanol, i-Propanol etc. aufgeführt werden. Geeignete Lösungsmittel-Gemische sind somit z.B. Cyclohexan/Ethanol, Petrolether/Isopropanol etc.

Die Erfindung wird nachstehend in größerem Detail an der Trennung des racemischen Gemisches von Ibuprofen erläutert, wobei die therapeutisch im Vordergrund stehende S(+)-Antipode in einfacher Weise in hoher Reinheit gewinnbar ist. Die im Zusammenhang hiermit erfolgten Angaben lassen sich in analoger Weise auf die anderen Vertreter der therapeutisch eingesetzten 2-Aryl-propionsäuren übertragen, weshalb die Erfindung nicht auf diese bevorzugte Ausführungsform des erfindungsgemäßen Verfahrens beschränkt ist:

Das diastereomere Salz aus S(+)-Ibuprofen und dem genannten Aminoalkohol fällt bei Raumtemperatur mit guten Ausbeuten aus einem Gemisch von Isopropanol und Petrolether 1:10 aus, während das Salz der R(-)-Form mit dem Aminoalkohol weitgehend in Lösung bleibt.

Das diastereomere Salz läßt sich durch eine Umkristallisation, z.B. aus einem Gemisch von Isopropanol und Petrolether von 1:1 - 5, so rein gewinnen, daß die nach dem Ansäuern mit Mineralsäure freigesetzte S(+)-Form des Ibuprofens mit einer optischen Reinheit von 95 bis 99% anfällt.

Aus dem kristallinen Salz läßt sich mit einer bei RT gesättigten schwachen Base bevorzugt N a$_2$CO$_3$-Lösung (pH 11) der Aminoalkohol kristallin abscheiden. Aus der anfallenden Lösung kann durch Versetzen mit Mineralsäure, z.B. 10N-Salzsäure, S(+)-Ibuprofen kristallin gewonnen werden.

Es wurde gefunden, daß das erfindungsgemäße Verfahren dadurch wirtschaftlich gestaltet werden kann, daß der Aminoalkohol in praktisch chemisch und optisch reiner Form zurückgewonnen wird und die in den Mutterlaugen verbleibenden R(-)- und S(+)-Säuren durch Racemisierung der weiteren Verwendung zugeführt werden können. Bei der Ausfällung des diastereomeren Salzes sowie bei dem Umkristallisieren dieses Salzes fallen nämlich Mutterlaugen an, die R(-)- und S(+)-2-Aryl-propionsäure und die Base enthalten. Nach einem schonenden Entfernen des Lösungsmittels (Petrolether, Isopropanaol) läßt sich der Rückstand z.B. mit wäßrigem Alkali, bevorzugt mit Kalilauge, in eine wäßrige Lösung überführen. Dabei scheidet sich ein weiterer Teil des Aminoalkohols in kristallisierter Form ab. Aus der abgetrennten Lösung fallen nach dem Ansäuern die beiden enantiomeren Säuren an. In diesem Säuregemisch - in dem bei Ibuprofen der Anteil der R(-)Form überwiegt - lassen sich ohne oder mit Zusatz geringer Mengen eines Katalysators bei An- oder Abwesenheit eines Lösungsmittels (vornehmlich Wasser) bei Temperaturen von 100 bis 250 °C Normal- bzw. erhöhtem Druck die beiden Säuren racemisieren. Es fällt damit ein Gemisch der beiden Säuren an, das in seiner Zu sammensetzung dem Ausgangsprodukt des racemischen Produktes, z.B. bei Ibuprofen, entspricht. Dieses Racemat läßt sich nach Zusatz des optisch aktiven Aminoalkohols erneut in den Kristallisationsprozeß zur Abtrennung der S-Form einführen.

Das Verfahren ist durch die Rückgewinnung der Base und der Racemisierung der zurückgebliebenen Komponente, z.B. des R(-)-Ibuprofens, wirtschaftlich durchführbar. Die Base läßt sich zu mehr als 80% zurückgewinnen ($[\alpha]_D^{20}$ : -28.0 ° bis - 30.5 °). Die Ausbeuten sind hoch, z.B. betragen sie an S(+)-Ibuprofen mit 95-99% optischer Reinheit (- $[\alpha]_D^{20}$ : +57,6 °) 95-100% des eingesetzten racemischen Ibuprofens. Das erfindungsgemäße Verfahren wird nachstehend anhand typischer, aber auch bevorzugter Ausführungsformen erläutert:

Beispiel 1:

1-3 mol (206.3 bis 618.9 g) racemisches Ibuprofen werden mit 1 mol (212.2 g) D(-)-threo-1-p-Nitrophenyl-2-aminopropan-1,3-diol in 420 bis 850 ml Isopropanol suspendiert und die Suspension erhitzt bis eine klare Lösung eingetreten ist. Zu dieser Lösung wird in der Wärme 2.2 bis 8.5 l Petrolether (Kp. 60-90°C) gegeben und der Ansatz so lange erwärmt bis eine klare Lösung eingetreten ist. Die Lösung wird mit oder ohne Rühren bis auf ca. 20-25°C (RT) abgekühlt. Es tritt Kristallisation ein. Die Kristalle werden rasche von der Mutterlauge abgetrennt (Ausbeute ca. 58-65 %, bezogen auf ein diastereomeres Salz (1:1) aus S(+)-Ibuprofen und dem Aminoalkohol).

Das kristalline Salz wird aus einem Gemisch aus Ispropanol und Petrolether 1:4 umkristallisiert: Ausbeute 80-85 %. Anschließend wird das Salz in einer wäßrigen gesättigten Natriumcarbonat-Lösung suspendiert. Es fällt der Aminoalkohol kristallin aus: Ausbeute 35-50 %, bezogen auf die eingesetzte Menge Aminoalkohol. Die Säure, die als Natriumsalz in Lösung vorliegt, läßt sich durch Ansäuern mit einer Mineralsäure kristallin mit einer optischen Reinheit von 95-99 % und einer Ausbeute von 80-85 %, bezogen auf das 1. Kristallisat, ausfällen.

Die Mutterlaugen, die bei der ersten Fällung des diastereomeren Salzes und bei dem Umkristallisieren dieses Salzes anfallen, werden vereinigt und von dem Lösungsmittelgemisch befreit (z.B. durch Destillation im Vakuum oder durch Wasserdampfdestillation). Der anfallende ölige Rückstand wird mit wäßriger Kalilauge versetzt. Es kristallisiert der Aminoalkohol aus (Ausbeute: 22-30 % der eingesetzten Menge). Die in Lösung gegangenen Säuren von S(+)- und R(-)-Ibuprofen lassen sich durch Ansäuern mit Mineralsäure kristallin abscheiden. Die getrockneten Säuren werden ohne oder mit einer geringen Menge Katalysator, z.B. Kaliumhydroxid oder Natriumibuprofenat, mit oder ohne Lösungsmittel (z.B. Wasser) bei Normaldruck oder erhöhtem Druck kurze Zeit auf Temperaturen zwischen 100-250°C erhitzt. Es tritt Racemisierung ein, durch die ein Gemisch von S(+)- und R(-)-Ibuprofen erhalten wird, das in der Zusammensetzung dem als Ausgangssubstanz eingesetzten racemischen Ibuprofen entspricht.

Dieses Racemat läßt sich nach Zusatz des optisch aktiven Aminoalkohols, der auch aus den Mutterlaugen gewonnen sein kann, erneut in den Auftrennungsgang einsetzen.

## Gesamtausbeute:

S(+)-Ibuprofen mit einer optischen Reinheit von 95-99 % $[\alpha]_D^{20}$ : +57.6) in einer Ausbeute von 95-98 % des eingesetzten Racemates.

Aminoalkohol ca. 80 % $[\alpha]_D^{20}$ : -28.0-30.5; Fp. 162-167, Zers.) der eingesetzten Menge.

## Beispiel 2:

1.0 mol (230.2 g) racemisches Naproxen und 0.5 mol (106.1 g) L-(+)-threo-1-p-Nitrophenyl-2-aminopropan-1,3-diol werden in 3.5 l Isopropanol in der Wärme gelöst und anschließend die Lösung 24 h bei 5°C gehalten. Es tritt Kristallisation ein: Ausbeute 152 g (= 68.72 % eines Salzes 1:1 aus Aminoalkohol und S(+)-Naproxen). Fp.: 167-169°C; optische Reinheit der in dem Salz enthaltenen Säure: 15.2 %.

Nach 2maligem Umkristallisieren des ausgefällten Salzes aus Isopropanol fällt das Salz in einer Ausbeute von 54.2 g an (= 24.5 % bezogen auf ein Salz 1:1 aus Aminoalkohol und S-(+)-Naproxen). Die in dem Salz enthaltene Säure besitzt eine optische Reinheit von 90 %.

50 g des diastereomeren Salzes werden in 500 ml Wasser suspendiert, 50 ml 2N Schwefelsäure zugegeben und 1/2 h bei Raumtemperatur gerührt: Die freigesetzte Säure fällt aus, Ausbeute 28.1 g (= 99.5 %); Fp. 152-155°C. Die Säure entspricht 24.38 % des S-(+)-Naproxens des racemischen Naproxens; die optische Reinheit der Säure beträgt 90.6 %.

Die Wiedergewinnung der Base erfolgt wie im Beispiel 1 beschrieben mit einer Ausbeute von 80-85 %; Fp. 163-166°C $[\alpha]_D^{20}$ : 28-30°. Das aus den Mutterlaugen freigesetzte Gemisch aus R-(-)-Naproxen und S-(+)-Naproxen wird racemisiert und erneut der Trennungsoperation zugeführt.

Gesamtausbeute: S-(+)-Naproxen 87-93 % in 90-95 %iger optischer Reinheit (bezogen auf das eingesetzte racemische Naproxen); Fp. 154-155°C $[\alpha]_D^{20}$ = +65.2.

L-(+)-threo-1-p-Nitrophenyl-2-aminopropan-1,3-diol: 75-82 % Ausbeute, Fp. = 163-166°C $[\alpha]_D^{20}$ = +28.0 - 29.3°.

## Beispiel 3:

1.0 mol (244.3 g) racemisches Flurbiprofen und 0.5 mol (106.1 g) D-(-)-threo-1-p-Nitrophenyl-2-aminopropan-1,3-diol werden in 350 ml Isopropanol in der Wärme gelöst und anschließend 2 l Petrolether (60-90°), der auf die Temperatur der Isopropanol-Lösung erwärmt wird, zugegeben. Der Ansatz wird 20 h bei Raumtemperatur gehalten. Es tritt Kristallisation ein: Ausbeute 195.0 g (= 67.65 % eines Salzes aus dem Aminoalkohol, S(+)-Flurbiprofen und Isopropanol (1:1:1)); Fp. 127-128°. Die optische Reinheit der in dem diastereomeren Salz enthaltenen Säure beträgt 10.2 %. Nach 2maligem Umkristallisieren des Salzes aus Ispropanol: Ausbeute 150.0 g (= 52.04 % eines Salzes aus Aminoalkohol, S(+)-Flurbiprofen und Isopropanol (1:1:1)); Fp. 130-132°, optische Reinheit der in dem Salz enthaltenen Säure 24.2 %. Erneute Kristallisation des Salzes aus Ethylacetat: Ausbeute 110 g (= 48.20 % bezogen auf ein Salz 1:1 aus Aminoalkohol und S-(+)-Flurbiprofen). Die optische Reinheit des S-(+)-Flurbiprofens beträgt 35 %. Wiederholtes Umkristallisieren ergibt eine Säure mit der optischen Reinheit von 90-95 %.

Nach bekannten Verfahren wird das diastereomere Salz zersetzt durch Zugabe von 2N Schwefelsäure, die Freisetzung der Säure verläuft mit quantitativen Ausbeuten.

Die Wiedergewinnung der Base erfolgt wie im Beispiel 1 aus dem diastereomeren Salz bzw. den Mutterlaugen. Aus dem gleichen Substrat wird das Säuregemisch (R(-)-Flurbiprofen und S-(+)-Flurbiprofen) isoliert und anschließend nach bekannten Verfahren racemisiert.

Gesamtausbeute: S-(+)-Flurbiprofen: 85-90 % des eingesetzten Racemats; Fp. 100-103°C; optische Reinheit: 90-95 % $[\alpha]_D^{20}$ = +43.85.

D-(-)-threo-1-p-Nitrophenyl-2-aminopropan-1,3-diol: 80-85 %, Fp. = 162-166°C $[\alpha]_D^{20}$ = +28-30°.

Beispiel 4:

1.0 mol (273.7 g) racemisches Carprofen und 0.5 mol (106.1 g) D-(-)-threo-1-p-Nitrophenyl-2-aminopropan-1,3-diol werden in 390 ml Aceton in der Wärme gelöst und anschließend die Lösung 5 h bei ca. 5°C gehalten. Es tritt Kristallisation ein: Ausbeute 140 g (= 57.63 % bezogen auf ein Salz (1:1) aus Aminoalkohol und S-(+)-Carprofen); Fp. 204-205°C; die in dem Salz enthaltene Säure besitzt eine optische Reinheit von 52 %.

140 g der erhaltenen kristallinen Verbindung werden aus 220 ml Aceton umkristallisiert (2 h bei 5°C). Ausbeute: 46.7 g (= 19.22 % bezogen auf ein Salz (1:1) aus Aminoalkohol und S-(+)-Carprofen). Fp. 209-210°C. Die in dem Salz enthaltene Säure besitzt eine optische Reinheit von 81.2 %; erneute Umkristallisation: 92-95 %.

Die anfallende Mutterlauge wird auf die Hälfte des Volumen eingeengt und 24 h auf 5° gekühlt. Das ausfallende Kristallisat wird abgetrennt und getrocknet: Ausbeute: 47.8 g (= 19.67 % bezogen auf ein Salz (1:1) aus dem Aminoalkohol und S-(+)-Carprofen);Fp. 208-209°C. Die in dem Salz enthaltene Säure besitzt eine optische Reinheit von 61.8 %. Erneute Umkristallisation 92-95 %.

50 g des diastereomeren Salzes werden in 500 ml Wasser bei Raumtemperatur suspendiert, die Suspension mit 50 ml 2N Schwefelsäure versetzt und ca. 30 min bei Raumtemperatur gerührt. Es scheiden sich weiße Kristalle ab, die getrocknet werden. Ausbeute: 28.0 g (= 99.5 % bezogen auf die saure S-Komponente des Salzes); Fp. 202-205°C. Die optische Reinheit der freigesetzten Säure ist 91.5 % (bezogen auf S-(+)-Carprofen).

Die Base wird wie in dem Beispiel 1 beschrieben aus dem diastereomeren Salz und den Mutterlaugen zurückgewonnen und das freigesetzte Gemisch aus R-(-)-Carprofen und dem Rest des S-(+)-Carprofens racemisiert. Sowohl die Base wie auch das racemische Carprofen lassen sich erneut in die Trennungsoperation einsetzen.

Gesamtausbeute:

S-(+)-Carprofen mit einer optischen Reinheit von 92-95 % $[\alpha]_D^{20}$ = +52.8°, ca. 95 % des eingesetzten Racemats; Fp. 202-205°C.
Aminoalkohol: Ausbeute: ca. 85 % $[\alpha]_D^{20}$ = -28.0-30.5.

Ansprüche

1. Verfahren zur Gewinnung von Enantiomeren von 2-Aryl-propionsäuren durch Umsetzung eines racemischen Gemisches derselben mit einem Amin-enantiomer, dadurch **gekennzeichnet,** daß man das Racemat der 2-Aryl-propionsäure mit einer optisch aktiven Form von threo-1-p-Nitrophenyl-2-aminopropan-1,3-diol in die diastereomeren Salze überführt, diese auftrennt und die so erhaltenen reinen Diastereomeren in die freien Säuren der enantiomeren Formen der 2-Aryl-propionsäure oder deren Salze überführt.

2. Verfahren nach Anspruch 1, dadurch **gekennzeichnet,** daß die 2-Aryl-propionsäure unter Flurbiprofen, Fenoprofen, Ketoprofen, Naproxen, Benoxaprofen und Ibuprofen ausgewählt ist.

3. Verfahren nach Anspruch 2, dadurch **gekennzeichnet,** daß man als 2-Aryl-propionsäure Ibuprofen einsetzt.

4. Verfahren nach Anspruch 1 oder 2, dadurch **gekennzeichnet,** daß man die diastomeren Salze durch Kristallisation auftrennt.

5. Verfahren nach einem oder mehreren der vorherigen Ansprüche, dadurch **gekennzeichnet,** daß man die 2-Aryl-propionsäure mit D(-)-threo-1-p-Nitrophenyl-2-aminopropan-1,3-diol umsetzt.

6. Verfahren nach einem oder mehreren der vorherigen Ansprüche, dadurch **gekennzeichnet,** daß man die nach Ausfällung des Salzes der optischen Antipode verbleibende Mutterlauge, gegebenenfalls unter Zufügen von Mutterlauge aus der Umkristallisierung der abgetrennten Antipode eingengt, mit wäßriger Alkalilauge aufschlämmt und hieraus den Aminoalkohol abscheidet.

7. Verfahren nach einem oder mehreren der vorherigen Ansprüche, dadurch **gekennzeichnet,** daß man die nach Abtrennung des ausgefällten Salzes der optischen Antipode und des Aminoalkohols gewonnene Lösung der Enantiomeren der 2-Aryl-propionsäure erneut racemisiert und in das Verfahren rückführt.

8. Verfahren nach einem oder mehreren der vorherigen Ansprüche, dadurch **gekennzeichnet,** daß man Ibuprofen als racemisches Gemisch einsetzt und hieraus S(+)-Ibuprofen gewinnt.